# EUROPEAN PATENT APPLICATION

(11) **EP 4 653 023 A1**
(43) Date of publication of application: **26.11.2025**
(21) Application number: 24744696.6
(22) Date of filing: 18.01.2024
(51) Int. Cl.: A61L 27/36

(54) **TRANSPLANT MATERIAL**

(30) Priority: 20.01.2023 JP 2023007159
(71) Applicant: BIOS Co., Ltd, Tokyo 110-0015 (JP)
(72) Inventor: KINOSHITA, Yoshitaka, Tokyo 110-0015 (JP); YOKOO, Takashi, Tokyo 108-0074 (JP); KOBAYASHI, Eiji, Wakayama-shi, Wakayama 640-8263 (JP)
(74) Representative: Cabinet Becker et Associés
(86) International application number: PCT/JP2024/001269
(87) International publication number: WO 2024/154775

(57) **Abstract**

A transplant material containing an aggregate of a plurality of renal primordia provided with urinary bladder primordia, wherein the plurality of renal primordia provided with urinary bladder primordia form an aggregate by joining via the respective urinary bladder primordia.

## Description

### [Technical Field]

The present invention relates to a transplant material. Priority is claimed on Japanese Patent Application No. 2023-007159, filed January 20, 2023, the content of which is incorporated herein by reference.

### [Background Art]

The only curative treatment for end-stage renal failure is renal allotransplantation. However, there is a worldwide shortage of donor organs, and an increasing number of patients are dying while awaiting a donor organ. Xenotransplantation is garnering much attention as a way of dealing with this donor shortage. In the field of xenotransplantation, research is progressing into suppressing rejection by genetically modifying the donor pig, and kidney transplants from genetically modified pigs into brain-dead humans, and heart transplants into patients with heart failure have already been conducted. However, even with animals that have undergone considerable gene editing, xenotransplantation still requires powerful immunosuppression, and complications such as infections and cancer remain a significant problem.

Embryonic organ transplantation is a method in which an embryonic organ is transplanted into an adult prior to the development of vascular structures, and then grown inside the body of the host. In embryonic organ transplantation, it is known that the blood vessels that develop are derived from the host, the transfer of antigen presenting cells is minimal, and the level of MHC antigen expression is lower than that of an adult organ, and therefore embryonic organ transplantation is known to offer an immunological advantage compared with adult organ transplantation.

In the case of kidneys, it is known that by transplanting embryonic metanephroi (renal primordia) into an adult body, urine production becomes possible. However, compared with adult kidneys, the smaller renal primordia have a much lower glomerular filtration rate, and only a short extension of life can be achieved compared with the case of no kidneys.

The inventors of the present invention have succeeded in growing transplanted embryonic metanephroi with good suppression of nephropathy caused by hydronephrosis, by transplanting renal primordia provided with urinary bladder primordia (see non-Patent Document 1).

### [Citation List]

### [Non-Patent Document]

[Non-Patent Document 1]
Yokote S, et al., Urine excretion strategy for stem cell-generated embryonic kidneys., Proc Natl Acad Sci U.S.A, 112, (42), 12980 to 12985, 2015.

### [Summary of Invention]

### [Technical Problem]

In order to increase renal function, a large number of renal primordia must be transplanted. Consequently, a technique for more efficiently reconstructing the urinary pathway would be very desirable. Accordingly, an object of the present invention is to provide a technique for efficiently reconstructing the urinary pathway.

### [Solution to Problem]

The present invention includes the following aspects.
[1] A transplant material containing an aggregate of a plurality of renal primordia provided with urinary bladder primordia, wherein the plurality of renal primordia provided with urinary bladder primordia form an aggregate by joining via the respective urinary bladder primordia.
[2] The transplant material according to [1], wherein the urinary bladder primordia are transplanted so as to connect to the ureter of the host.
[3] The transplant material according to [2], wherein the host is a cat.
[4] The transplant material according to [2], wherein the host is a human.
[5] The transplant material according to [1], wherein the renal primordia provided with urinary bladder primordia are derived from wild pig embryos.

### [Advantageous Effects of Invention]

The present invention is able to provide a technique for efficiently reconstructing the urinary pathway.

### [Brief Description of the Drawings]

FIG. 1 is a schematic illustration of a renal primordia aggregate in an embodiment of the present invention.
FIG. 2 shows a diagram and a photograph of an aggregate cloaca produced from SD rat renal primordia with urinary bladder primordia having an introduced GFP gene (with Tg, GFP⁺) and SD rat renal primordia with urinary bladder primordia without an introduced GFP gene (no Tg, GFP⁻).
FIG. 3 is a series of photographs of an aggregate cloaca three weeks after transplantation into an immunodeficient mouse.
FIG. 4 is a series of immunostaining images of tissue sections three weeks after transplantation of an aggregate cloaca into an immunodeficient mouse.
FIG. 5 shows a diagram and a photograph of five aggregate cloacae produced from LEW/SsNS1c rat renal primordia with urinary bladder primordia in a similar manner to Test Example 1 and then transplanted into the retroperitoneal cavity of an adult LEW/SsNS1c rat.
FIG. 6 includes a photograph on the left illustrating the state upon performing a second laparotomy three weeks after transplantation of the aggregate cloacae, and a photograph on the right taken following resection of the left kidney and reconstruction of the urinary pathway.
FIG 7 is a photograph taken following resection of the right kidney of the recipient 10 weeks after transplantation of the aggregate cloacae.
FIG. 8 is a series of graphs illustrating the results of measuring the bodyweight, measuring the urine volume, food intake and water intake using a metabolic cage, and conducting blood tests following resection of both recipient kidneys from rats transplanted with aggregate cloacae.
FIG. 9 is a series of photographs taken following the transplantation of two aggregate cloacae produced using pig renal primordia provided with urinary bladder primordia into the left retroperitoneal cavity of an immunosuppressed pig.
FIG. 10 is a series of photographs taken following urinary pathway reconstruction four weeks after the transplantation of the aggregate cloacae.

### [Description of Embodiments]

### [Transplant Material]

In one embodiment, the present invention provides a transplant material containing an aggregate of a plurality of renal primordia provided with urinary bladder primordia, wherein the plurality of renal primordia provided with urinary bladder primordia form an aggregate by joining via the respective urinary bladder primordia.

The term renal primordia describes embryonic stage kidneys, and the renal primordia of mammals are metanephroi. In an embryonic stage mammal, a structure composed of the urinary bladder priomordium, the ureter and the metanephroi forms as a single body, and in this description, this body is described as renal primordia provided with a urinary bladder primordium. The inventors of the present invention have named this embryonic stage urinary bladder priomordium - ureter - metanephroi structure a cloaca. In other words, in this description, renal primordia provided with a urinary bladder primordium may also be referred to as a cloaca. When a cloaca is transplanted into the retroperitoneal cavity of an animal, blood vessels from the recipient infiltrate the cloaca, development of the cloaca continues, and urine is produced.

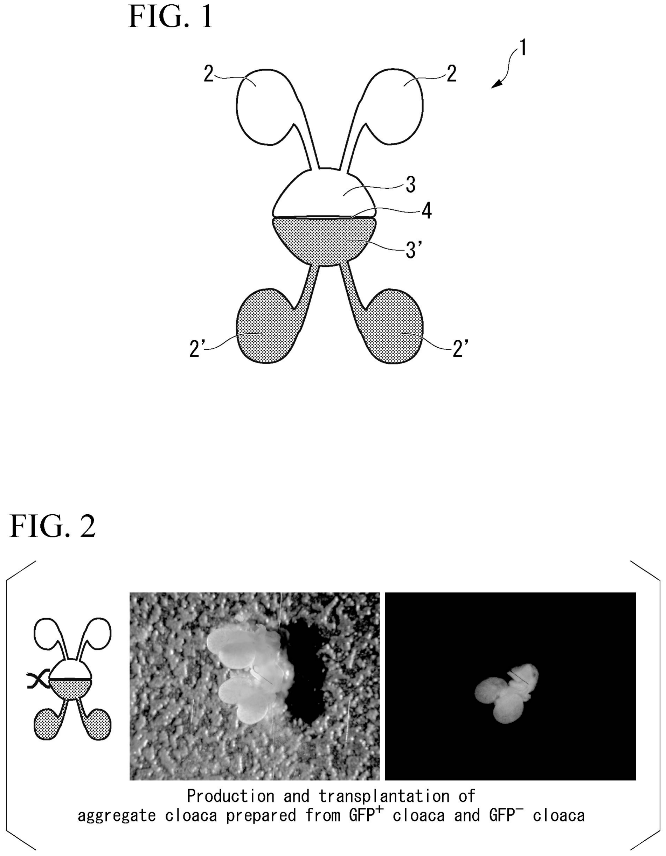

FIG. 1 is a schematic illustration of a transplant material according to an embodiment of the present invention. The transplant material of this embodiment contains an aggregate of a plurality of renal primordia provided with urinary bladder primordia (hereinafter also referred to as an "aggregate cloaca"). The aggregate 1 of renal primordia provided with urinary bladder primordia is an aggregate of a plurality of renal primordia 2 provided with urinary bladder primordia 3. The renal primordia 2 with a urinary bladder primordium 3 is joined to the renal primordia 2' with a urinary bladder primordia 3' via the urinary bladder primordia 3 and the urinary bladder primordia 3'. In other words, the aggregate 1 of renal primordia provided with urinary bladder primordia has a joint section 4.

There are no particular limitations on the method use for joining the plurality of renal primordia provided with urinary bladder primordia, and examples include methods in which the urinary bladder primordia of the plurality of renal primordia with urinary bladder primordia are cut, and the cross-sections of the urinary bladder primordia are then fused together, and methods in which a cannula is used to puncture and then link the urinary bladder portions of the renal primordia provided with urinary bladder primordia.

There are no particular limitations on the number of renal primordia provided with urinary bladder primordia used in constructing the aggregate of renal primordia with urinary bladder primordia, provided the number is a multiple, and the number may be two, three, or four or greater. By using an aggregate of a plurality of renal primordia provided with urinary bladder primordia, the urinary bladder capacity in the aggregate increases, and the effect of the invention in suppressing hydronephrosis is enhanced. Further, by simultaneously transplanting aggregates of a plurality of renal primordia provided with urinary bladder primordia, the number of anastomotic sites required during reconstruction of the urinary pathway can be dramatically reduced.

Further, the transplant material may contain a plurality of aggregates each having a plurality of renal primordia provided with urinary bladder primordia. There are no particular limitations on the number of aggregates of renal primordia provided with urinary bladder primordia contained within the transplant material, and the number may be one, two, three, four, five, six, or seven or more. By increasing the number of aggregates of renal primordia provided with urinary bladder primordia, the amount of renal tissue contained in the overall transplant material is increased, and the renal function is enhanced.

The animal from which the renal primordia provided with urinary bladder primordia are derived is preferably a mammal, and examples include hoofed animals such as cows, sheep, pigs, goats and horses, and rodents such as hamsters, marmots, rats and mice. Among these, pigs, rats and mice are preferred.

The renal primordia with urinary bladder primordia are preferably derived from pig embryos at four to seven weeks (28 to 49 days) of embryonic development. Pig embryos at 45 days of embryonic development are of substantially the same size as human fetuses at 16 weeks of embryo development. By using renal primordia derived from pig embryos within the above development range, growth into renal tissue that produces urine without differentiation into other tissue tends to occur more readily.

The renal primordia provided with urinary bladder primordia are preferably derived from wild pig embryos. In the present invention, genetically modified pigs are not required, and aggregates of renal primordia with urinary bladder primordia from clean pigs can be used favorably. Accordingly, the present invention is not limited by animal genetic modification restrictions. Moreover, in the present invention, large amounts of immunosuppressants are not required, and the host need not be subjected to powerful immunosuppression.

Furthermore, the renal primordia provided with urinary bladder primordia may also be derived from genetically modified non-human animals provided with a system for conditional removal of at least a portion of the renal primordia with urinary bladder primordia, such as genetically modified non-human animals capable of inducing programmed cell death in a variable manner. For example, by removing a portion of a transplanted renal primordia provided with urinary bladder primordia, while also injecting exogenous nephron progenitor cells into the renal primordia with urinary bladder primordia, the transplanted renal primordia provided with urinary bladder primordia can be substituted with the allogenic cells of the recipient.

Examples of the recipient into which the transplant material of an embodiment of the present invention is transplanted include humans, monkeys, cows, sheep, pigs, goats, horses (and particularly race horses), dogs, cats, rabbits, hamsters, marmots, rats and mice, although humans and cats are preferred. For example, in cat medical practice, which has low xenogeneic restrictions, a transplant material of an embodiment of the present invention using renal primordia with urinary bladder primordia derived from wild pig embryos can be used in the treatment of cats with renal failure. Moreover, the results of clinical tests in cats can be used to verify the safety in humans.

### [Transplantation Method]

In one embodiment, the present invention provides a kidney transplantation method including a step (a) of transplanting a transplant material containing an aggregate of a plurality of renal primordia provided with urinary bladder primordia, wherein the plurality of renal primordia provided with urinary bladder primordia form an aggregate by joining via the respective urinary bladder primordia, into the body of a patient or affected animal, and a step (b) of connecting the urinary bladder primordia with the ureter of the recipient a prescribed period after the transplantation. Here, the recipient refers to the patient or affected animal, wherein examples of the patient or affected animal include humans and cats.

The method of this embodiment may also be referred to as a treatment method for kidney disease. The transplant material described above can be used as the transplant material. In other words, in one embodiment, the present invention provides a treatment method for kidney disease, the treatment method including a step (a) of transplanting a transplant material containing an aggregate of a plurality of renal primordia provided with urinary bladder primordia, wherein the plurality of renal primordia provided with urinary bladder primordia form an aggregate by joining via the respective urinary bladder primordia, into the body of a patient or affected animal, and a step (b) of connecting the urinary bladder primordia with the ureter of the recipient a prescribed period after the transplantation.

Leaving the transplant material transplanted in the above step (a) for a prescribed period allows the transplant material to grow and urine production to start. This prescribed period may be any period that enables satisfactory growth of the transplant material, but ends before hydronephrosis occurs. The prescribed period may be adjusted appropriately in accordance with the symptoms and the like of the patient or affected animal, and for example, may be within a range from 1 to 10 weeks.

Subsequently, once the prescribed period from transplantation has elapsed, step (b) is conducted. **In** step (b), the urinary bladder primordia of the transplant material (the grown urinary bladder primordia) are connected to the ureter of the patient or affected animal. Examples of the connection method include end-to-end anastomosis, end-to-side anastomosis, side-to-side anastomosis and side-to-end anastomosis, and of these, end-to-side anastomosis is preferred.

By connecting the urinary bladder primordia of the transplant material and the urinary bladder of the patient or affected animal via the ureter of the patient or affected animal, urine produced by the transplant material can be excreted into the urinary bladder of the patient or affected animal. More specifically, urine produced by the kidneys of the transplant material is excreted to the urinary bladder primordia of the transplant material, and then passes from the urinary bladder primordia of the transplant material, through the ureter of the patient or affected animal, and is excreted into the urinary bladder of the patient or affected animal.

Because the method of this embodiment is a method for transplanting a differentiation stage embryonic organ, the progenitor cells proliferate, differentiate and mature after step (a). By utilizing this feature, and transplanting exogenous cells into the renal primordia, kidney tissue derived from the exogenous cells can be grown. By using this technique, it is possible to reduce immunogenicity by bringing cells inside the organ closer to the recipient.

### [Examples]

The present invention is described below using a series of examples, but the present invention is not limited by the following examples.

### [Method]

### (Anesthesia and Euthanasia of Mice and Rats)

Operations on mice and rats were conducted under general anesthesia with isoflurane (5% to induce anesthesia, and 1 to 3% to maintain anesthesia) and analgesia using 1.0 mg/kg subcutaneous administration of butorphanol, and following a laparotomy, either a cesarean section was performed followed by euthanasia by exsanguination, or in the case of a recipient, transplantation of the transplant material was conducted, followed by reconstruction of the urinary pathway and a bilateral nephrectomy. Recipients were given postoperative analgesia using 1.0 mg/kg subcutaneous administration of butorphanol and 10 mg/kg subcutaneous administration of enrofloxacin, and kept warm until wakening.

### (Anesthesia and Euthanasia of Pigs)

Pigs were placed under fasting conditions 12 hours prior to surgery (drinking water was permitted), and following analgesia and sedation by administration with 0.02 mg/kg of medetomidine, 0.2 mg/kg of midazolam and 0.02 mg/kg buprenorphine, were subjected to a laparotomy under general anesthesia with isoflurane (5% to induce anesthesia, and 1 to 3% to maintain anesthesia), and then either a cesarean section was performed, or in the case of a recipient, transplantation of the transplant material was conducted, followed by reconstruction of the urinary pathway and a bilateral nephrectomy. Following surgery, the pigs were kept warm until wakening, and 0.02 mg/kg of buprenorphine was then administered twice daily for three days, and 30 mg/kg of cefazolin was administered twice daily for 7 days.

### [Test Example 1]

### (Confirmation of Urinary Bladder Fusing)

Pregnant female SD-Tg (CAG-EGFP) rats (from Sankyo Labo Service Corporation, Inc.) at day 16 of pregnancy, and 6 to 8 week-old male NOD/Shi-scid IL-2Ry KO Jic mice (from CLEA Japan, Inc.) were used.

The embryos were extracted from the female SD-Tg (CAG-EGFP) rats at day 16 of pregnancy, separated into those individuals with Tg (GFP⁺) and those without Tg (GFP⁻), and the renal primordia with urinary bladder primordia were resected. The urinary bladder primordia of the renal primordia with urinary bladder primordia derived from the embryos with Tg (GFP⁺ cloaca) and the urinary bladder primordia of the renal primordia with urinary bladder primordia derived from the embryos without Tg (GFP⁻ cloaca) were cut and joined by suturing with 11-0 nylon thread so that the cut cross-sections made contact with each other, thus producing aggregates of the renal primordia with urinary bladder primordia (aggregate cloaca). The joined urinary bladder primordia fused in vivo so as to share a common lumen. An aggregate cloaca produced from renal primordia with urinary bladder primordia with Tg and without Tg is illustrated in FIG. 2.

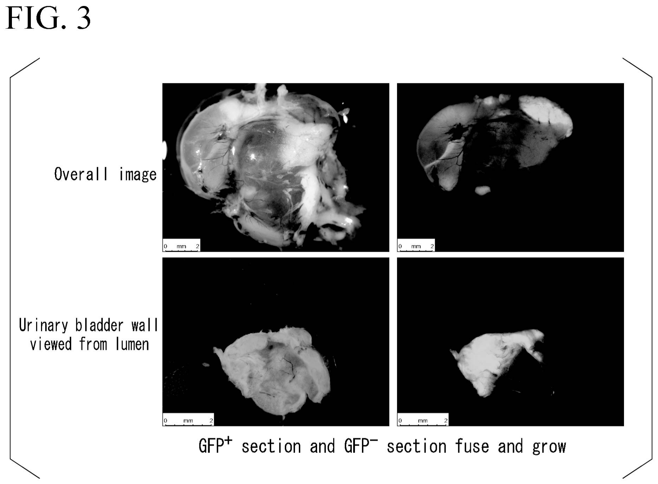

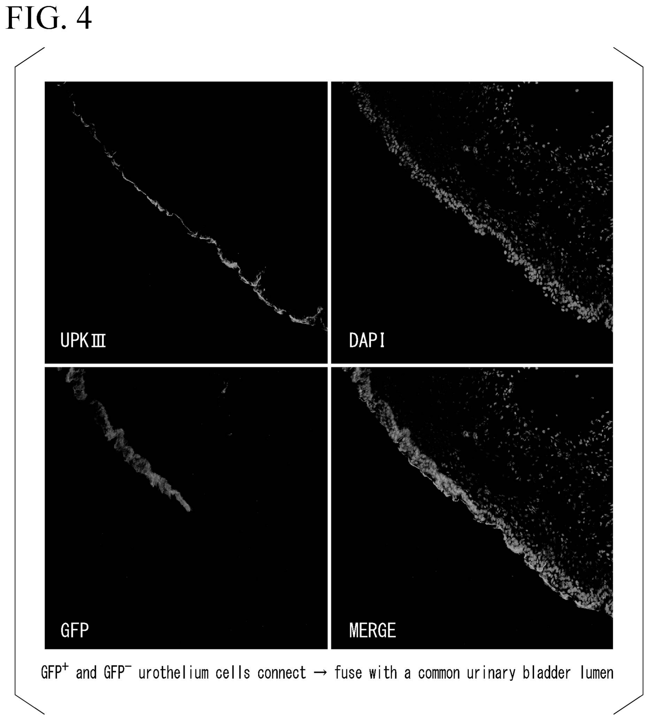

Subsequently, the prepared aggregate cloaca were transplanted into the left retroperitoneal cavity of adult NOD/Shi-scid IL-2Rγ KO Jic mice. As illustrated in FIG. 3, retention of urine was confirmed three weeks after the transplantation. The aggregate cloaca were collected three weeks after transplantation and subjected to immunostaining. The results of the immunostaining are illustrated in FIG. 4. The results confirmed that GFP-positive cells and GFP-negative cells had connected to form urothelium stained with UPK3.

### [Test Example 2]

### (Confirmation of Long-Term Survival of Syngeneic Rats by Transplantation of Plurality of Aggregate Cloacae)

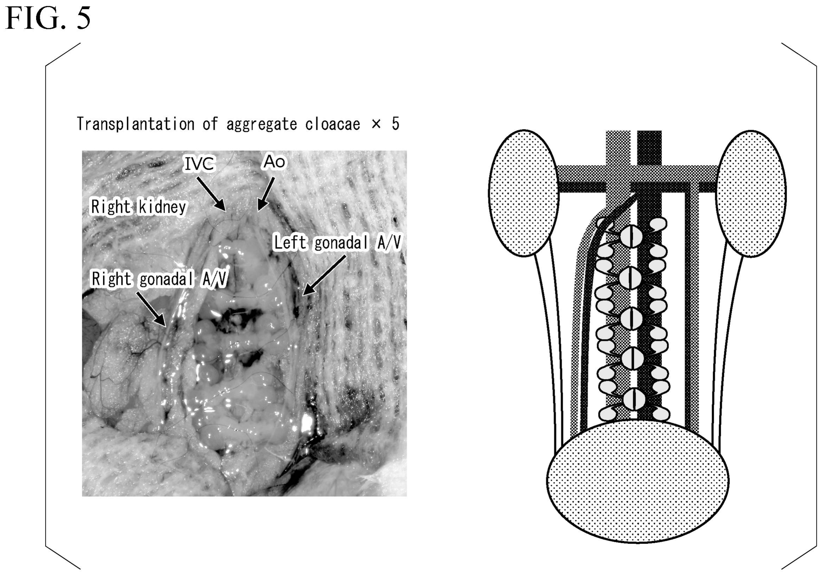

Pregnant female LEW/SsNSlc rats at day 17 of pregnancy and 8 week-old male LEW/SsNSlc rats (from Sankyo Labo Service Corporation, Inc.) were used. Renal primordia with urinary bladder primordia were resected from the embryos resected from the LEW/SsNSlc rats on day 17 of pregnancy, and aggregate cloacae were prepared in the same manner as Test Example 1. Subsequently, as illustrated in FIG. 5, five of these aggregate cloacae were transplanted into the retroperitoneal cavity of each adult LEW/SsNSlc rat.

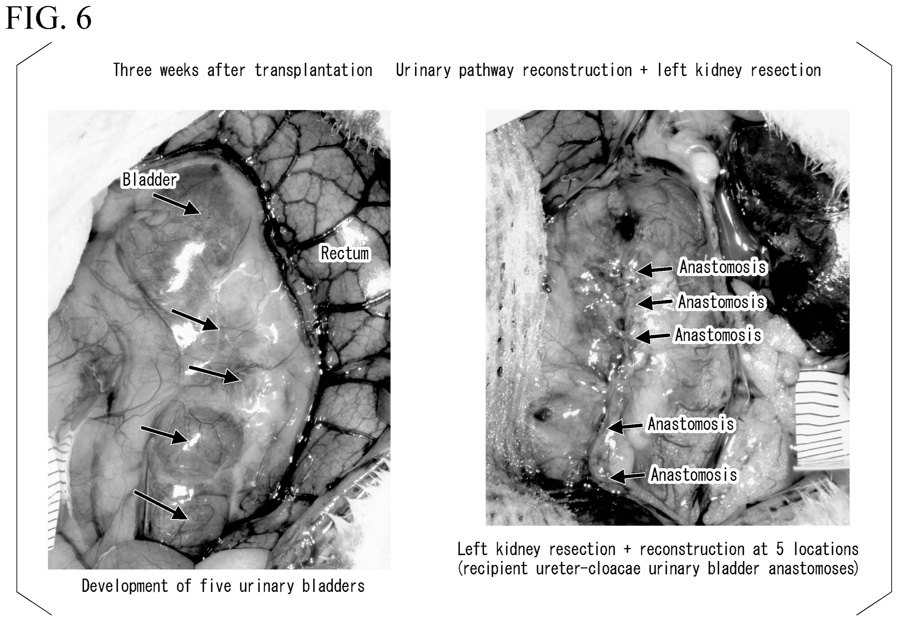

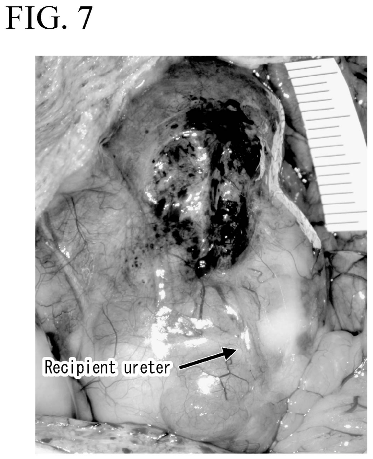

After three weeks, another laparotomy was performed. The left photograph in FIG. 6 illustrates the state following the laparotomy. Next, the left kidney was resected, and the ureter was cut at the renal hilum. Urine retention in the urinary bladder (the grown urinary bladder primordia) of the transplanted aggregate cloacae was confirmed. Subsequently, as illustrated in the right photograph of FIG. 6, the bladders derived from the five transplanted aggregate cloacae were each anastomosed to the left ureter of the recipient, and five drainage pathways to the outside of the body were reconstructed. Subsequently, as illustrated in FIG. 7, the right kidney of the recipient was resected 10 weeks after transplantation.

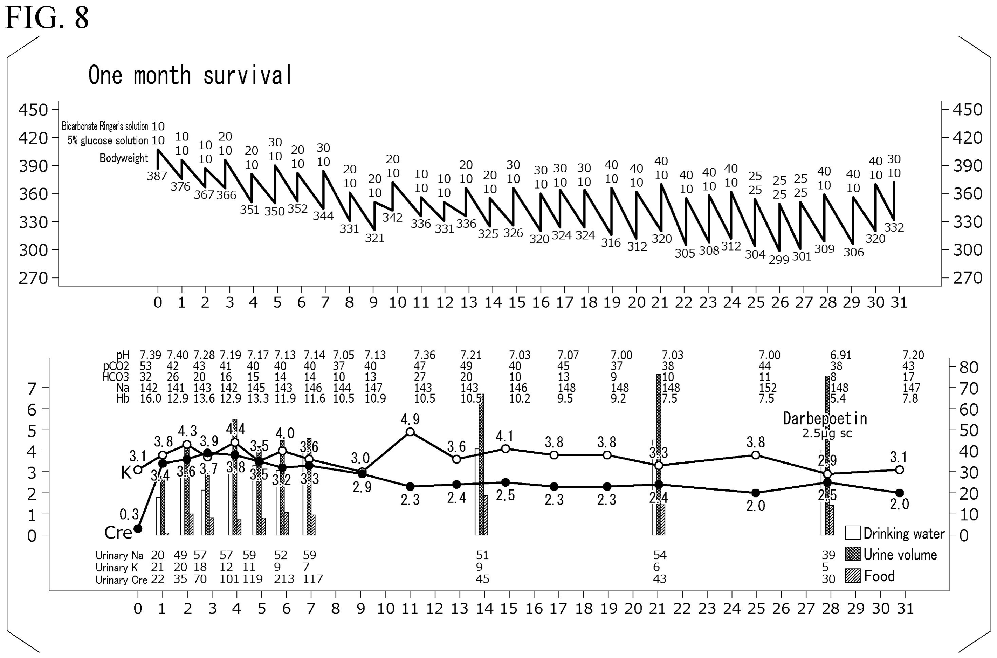

The survival period following the above procedure was then confirmed. Blood tests were conducted as required. Further, urine volume, food intake, and water intake were also measured in a metabolic cage. FIG. 8 illustrates the measurement results for bodyweight, urine volume, food intake, and water intake. In order to prevent hyperkalemia, the rats were given a low-potassium diet, and in those cases where hypokalemia developed, a normal diet CE-2 was given for several days. Further, bicarbonate Ringer's solution and a 5% glucose solution were also injected subcutaneously to correct electrolytes and acidosis. Moreover, darbepoetin was also administered subcutaneously to treat renal anemia. The results confirmed a survival period of at least one month.

### [Test Example 3]

### (Clinical Effects of Aggregate Grafts in Allogeneic Pigs)

Micro mini pigs (from Fuji Micra, Inc.) including a pregnant female at day 30 of pregnancy and a male aged 13 to 14 months were used. Renal primordia with urinary bladder primordia were resected from the embryos resected from the pregnant pig on day 30 of pregnancy, and after freezing and storing as previously reported, the renal primordia with urinary bladder primordia were thawed, and aggregate cloacae were prepared in the same manner as Test Example 1 and used for transplantation.

Twelve days prior to transplantation, a gastrostomy and a central venous catheter were placed in the recipient adult pig aged 13 to 14 months. Administration of immunosuppressants (2 mg/kg/day tacrolimus, target trough value 20 to 30 ng/mL; 1000 mg/body/day of mycophenolate mofetil; and 1 mg/kg/day of prednisolone) was started two days before transplantation.

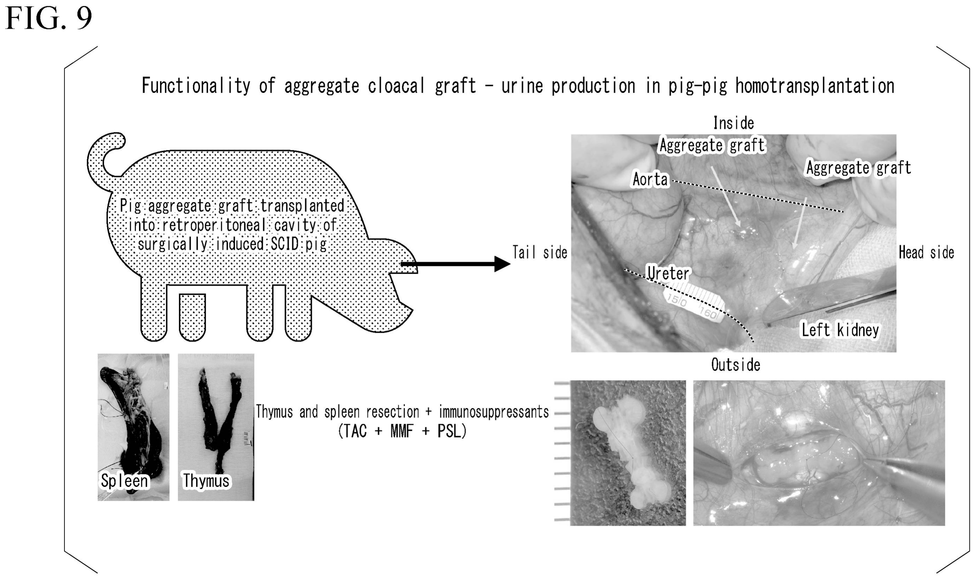

As illustrated in FIG. 9, on the day of transplantation, an incision was made in the neck of the recipient adult pig and the thymus was resected. Further, the spleen was also resected via an abdominal midline incision. Following this surgically induced SCID by resection of the thymus and spleen, two aggregate cloacae were transplanted into the left retroperitoneal cavity of the recipient adult pig.

Subsequently, after four weeks had elapsed, urinary pathway reconstruction (a left nephrectomy, and two anastomoses between the left ureter and the bladder derived from the aggregate cloacae) was performed, and a ureteral catheter was installed to guide urine derived from the transplanted aggregate cloacae (hereinafter sometimes referred to as the "aggregate cloacal graft" or "aggregate graft") to an external negative pressure bag. A 2.5 mm ameroid constrictor was placed in the renal artery of the right kidney, and was gradually narrowed to reduce renal function.

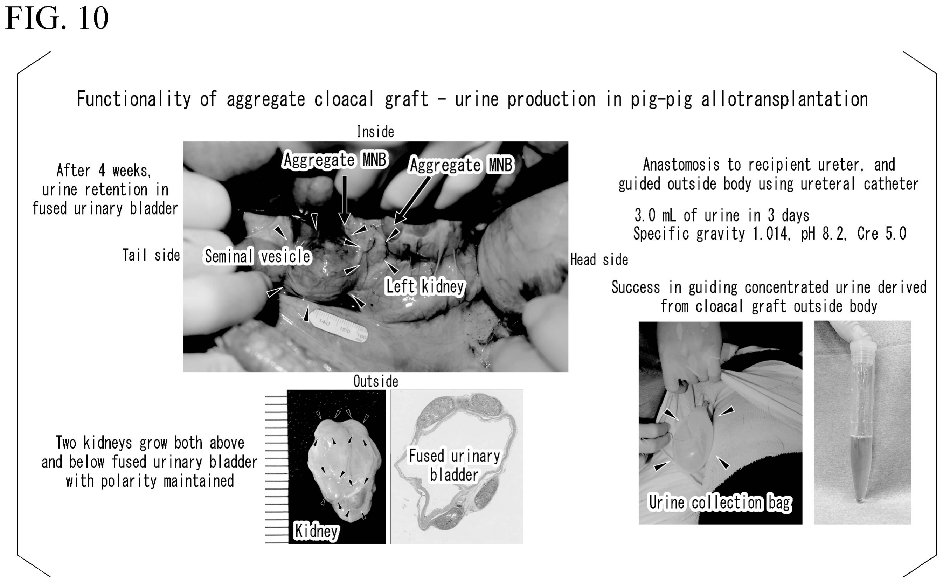

In the pathology conducted in the fourth week following transplantation, two kidneys had grown above and two kidneys below the fused bladder, indicating that bladder fusion also occurs in pigs. As illustrated in FIG. 10, following the urinary pathway reconstruction conducted four weeks after transplantation, the production of 3 mL of embryonic kidney-derived urine (specific gravity: 1.014, pH 8.2, creatinine (Cre): 5.0 mg/dL) was confirmed over a three day period.

In this test, an adult animal was successfully kept alive for one month using only the renal function derived from the embryonic kidneys. This provides new options for xenotransplantation therapies for renal failure that offer the possibility of achieving a reduction in the administration of immunosuppressants compared with vascularized transplants of adult organs.

### [Industrial Applicability]

The present invention is able to provide a technique for efficiently reconstructing the urinary pathway.

### [Reference Signs List]

- 1: Aggregate of renal primordia provided with urinary bladder primordia
- 2, 2': Renal primordia
- 3,3': Urinary bladder primordia
- 4: Joint section

## Claims

1. A transplant material comprising an aggregate of a plurality of renal primordia provided with urinary bladder primordia, wherein the plurality of renal primordia provided with urinary bladder primordia form an aggregate by joining via the respective urinary bladder primordia.

2. The transplant material according to Claim 1, wherein the urinary bladder primordia are transplanted so as to connect to the ureter of a host.

3. The transplant material according to Claim 2, wherein the host is a cat.

4. The transplant material according to Claim 2, wherein the host is a human.

5. The transplant material according to Claim 1, wherein the renal primordia provided with urinary bladder primordia are derived from wild pig embryos.
